(19) Europäisches Patentamt
European Patent Office
Office européen des brevets

(11) Numéro de publication: **0 209 462 A1**

(12) # DEMANDE DE BREVET EUROPEEN

(21) Numéro de dépôt: **86401564.9**

(22) Date de dépôt: **15.07.86**

(51) Int. Cl.⁴: **A 61 K 9/10**, A 61 K 31/10, A 01 N 25/04

(30) Priorité: **17.07.85 FR 8510927**

(43) Date de publication de la demande: **21.01.87 Bulletin 87/4**

(84) Etats contractants désignés: **AT BE CH DE GB IT LI LU NL SE**

(71) Demandeur: **SOCIETE NATIONALE ELF AQUITAINE**
**Société anonyme dite, Tour Elf 2, Place de la Coupole La Défense 6, F-92400 Courbevoie (FR)**
Demandeur: **SANOFI, 40, Avenue George V, F-75008 Paris (FR)**

(72) Inventeur: **Gautier, Jean-Claude, 5 Avenue Jean Jaurès, F-64140 Billere (FR)**
Inventeur: **Komornicki, Jacques, 12 Lotissement Curt, F-64230 Lescar (FR)**
Inventeur: **Foix, Jean, 9 rue de l'Yser, F-78200 Mantes la Jolie (FR)**
Inventeur: **Pastor, Gilbert, 103 La Cadoule, F-34160 Castries (FR)**

(74) Mandataire: **Kohn, Armand, 5 Avenue Foch, F-92380 Garches (FR)**

(54) **Composition liquide, stable, anthelmintique et fongicide, à base de bithionol sulfoxyde.**

(57) Composition anthelminthique et fongicide comprenant le bithionol sulfoxyde en tant que principe actif; ce sulfoxyde se trouve à l'état de microémulsion dans un liquide renfermant de l'eau, du solvant, du bithionol sulfoxyde et un ou plusieurs agents tensioactifs.

EP 0 209 462 A1

Composition liquide, stable, anthelminthique et fongicide, à base de bithionol sulfoxyde

L'invention concerne une composition de bithionol sulfoxyde sous une forme nouvelle permettant diverses applications thérapeutiques et agricoles de ce composé, plus vastes et efficaces qu'antérieurement. Elle comprend notamment des compositions liquides, stables, pouvant contenir - en association avec le sulfoxyde de bithionol - un dérivé de la tétramisole lévogyre.

Le composé, connu dans le commerce sous la dénomination de bithionol sulfoxyde, "BTS" ou "Bitin-S" est l'oxyde de bithionol, c'est-à-dire le corps :

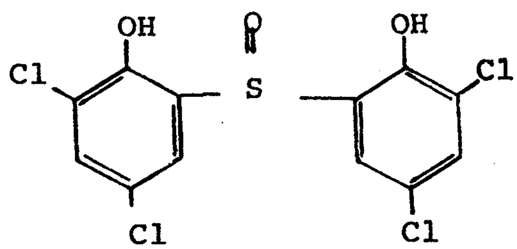

ou bis(hydroxy-2 dichloro-3,5 phényl) sulfoxyde. Ce produit est connu comme agent antiparasitaire et fongicide : il est employé avec succès en thérapeutique, en particulier celle des animaux. Ainsi, est-il couramment employé comme anthelminthique pour le traitement de la douve du foie, surtout dans l'élevage d'ovins et de bovins. Cependant, étant donné l'insolubilité dans l'eau de ce produit, (4 ppm à 25°C), la seule forme d'administration consistait jusqu'à présent à donner à l'animal une suspension aqueuse de ce médicament. Il fallait pour cela l'intervention de spécialistes, ou du moins de personnes très expérimentées, parce que la sédimentation des particules fines de l'anthelminthique dans leur suspension fausse le dosage et peut conduire accidentellement à un sous- ou surdosage. Dans le premier cas, l'efficacité du médicament étant compromise,

il y a des risques de mortalité du cheptel du fait de la maladie ; dans le second cas, on risque des intoxications plus ou moins graves. Bien que le bithionol sulfoxyde soit plus ou moins soluble dans différents solvants, on ne pouvait pas en envisager l'administration par voie parentérale à cause des réactions possibles de ces solvants qui ne répondaient pas aux impératifs d'inocuité requis en thérapeutique vétérinaire ; certains d'entre eux auraient pu convenir à condition d'être dilués avec de l'eau, mais cette solution ne pouvait pas être appliquée, parce qu'une dilution aqueuse fait reprécipiter le produit actif.

Pour les mêmes raisons, il était impossible d'obtenir une solution homogène d'un mélange de bithionol sulfoxyde avec un strongylicide.

La présente invention apporte un progrès marqué dans les traitements des atteintes justiciables du bithionol sulfoxyde et des mélanges de ce composé avec d'autres anthelminthiques. Elle permet l'application des composés actifs sous la forme d'un liquide homogène, ne donnant pas lieu à la sédimentation, susceptible par conséquent d'être dosé avec précision et appliqué par des personnes non spécialisées.Un autre avantage considérable de la composition suivant l'invention est la possibilité de l'administration de celle-ci par injection, forme qui était complètement exclue jusqu'à présent. Un autre avantage également très important de la composition selon l'invention est qu'elle permet l'utilisation du bithionol sulfoxyde et des autres anthelminthiques, composant le mélange, à des doses moindres que celles qui étaient jusqu'alors utilisées. Pour n'en donner qu'un exemple pour le bithionol sulfoxyde, cette dose peut passer de 40 mg/kg de poids vif traité à 30 mg/kg pour une même efficacité. Lorsque l'on sait que la dose maximale tolérée est de 150 mg/kg, cela fait passer l'indice thérapeutique de

moins de 4 à 5.

Sous cette nouvelle forme de liquide homogène, stable, les propriétés fongicides du bithionol sulfoxyde peuvent être mises à profit non seulement en ce qui concerne l'homme ou les animaux, y compris en aquaculture, mais également vis-à-vis des plantes. Il devient en effet possible de pulvériser le liquide ou solution suivant l'invention sur les plantes dont les feuilles absorbent alors le principe actif, au lieu de le laisser sous la forme d'une poudre sur leur surface :on gagne ainsi considérablement en activité. Ces nouvelles compositions sont également utilisables en aquaculture (eau salée et eau douce) et en pisciculture (eau douce) ce qui n'était pas possible jusqu'à maintenant, étant donné l'insolubilité dans l'eau de ces produits. Ce sont alors les parasites vivant à l'état libre dans l'eau, ou fixés sur l'hôte, qui sont tués.

La nouvelle forme des compositions suivant l'invention est une dispersion micellaire du composé en question dans un milieu aqueux renfermant un solvant du bithionol sulfoxyde, ainsi qu'un agent tensioactif. Ces dispersions présentent les caractères des micro émulsions et ont pratiquement l'aspect extérieur de solutions vraies. Elles jouissent d'une stabilité très convenable à des températures ne dépassant pas 70°C et surtout entre 0 et 60°C, ce qui veut dire qu'à ces températures, les liquides suivant l'invention peuvent être conservés indéfiniment, sans qu'il y ait sédimentation de bithionol sulfoxyde ou d'un autre constituant.

De préférence, la composition suivant l'invention contient également un agent de neutralisation de l'acidité ou/et un anesthésique local, ce qui, à la fois, rend son application indolore et améliore sa stabilité au stockage.

Dans une forme d'exécution préférée, la composition liquide renferme en outre un ou plusieurs autres produits actifs, pour augmenter le spectre d'activité du bithionol sulfoxyde. De tels produits sont des tétramisoles, c'est-à-dire tétrahydro-2,3,5,6 phényl-6 imidazo(2,1-b)thiazole

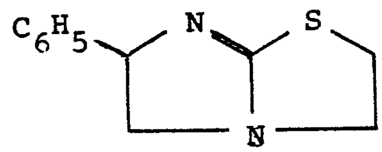

DL, D(+), L(-) et leurs sels, en particulier chlorhydrates. Parmi ces thiazoles, le levamisole, c'est-à-dire la forme L(-), et surtout son chlorhydrate constituent un anthelminthique et un immuno-potentialisateur très apprécié. Le chlorhydrate de L(-) levamisole est connu dans le commerce sous différents noms, entre autres comme "Ergamisol", "Levasole", "Némicide", "Solaskil" etc. Il peut être très avantageusement incorporé dans les compositions suivant l'invention, d'autant plus que sa solubilité dans l'eau est de l'ordre de 20%.

Ainsi, une composition particulièrement utile en thérapeutique animale est-elle constituée par une microémulsion de bithionol sulfoxyde renfermant du chlorhydrate de levamisole.Bien entendu d'autres dérivés du thiazole sus-indiqué peuvent faire partie de la composition suivant l'invention, à côté du bithionol sulfoxyde ; tel est par exemple le cas du dl tétramisole ; cependant, les formes L(-) sont en général plus efficaces.

Lorsque la composition suivant l'invention contient du chlorhydrate de levamisole ou d'un autre composé tétramisolique, il est important d'y inclure également un tampon de pH pour en assurer la stabilité ; le pH préféré est alors compris entre 2 et 5, ce qui concilie la stabilité du chlorhydrate de levamisole avec celle du bithionol sulfoxyde. Dans ces cas, il est souhaitable d'ajouter un anesthésique local pour contrebalancer l'effet d'un milieu un peu trop acide.

D'une façon générale, les compositions stables, liquides et homogènes, suivant l'invention, contenant éventuellement un dérivé thiazolique mentionné plus haut, présentent les teneurs suivantes, % en poids

| | En général | Préférées |
|---|---|---|
| Bithionol sulfoxyde | 1 à 25 | 5 à 20 |
| Composé thiazolique | 0 à 10 | 2-8 |
| Solvant du bithionol sulfoxyde | 1 à 50 | 10-35 |
| Agent tensioactif | 1 à 40 | 5-35 |
| Neutralisant | 1 à 15 | 3-10 |
| Eau tamponnée | 5 à 50 | 10-40 |
| Anesthésique local | 0,5 à 1 | 0,5 à 1 |

Lorsqu'on veut appliquer la technique connue à la préparation de compositions de bithionol sulfoxyde, on ne peut pas dépasser une concentration de 2%, ce qui conduit à l'emploi de quantités excessives pour injection anti-douvique.

Pour l'utilisation en aquaculture, on peut ajouter à la formule un anti-mousse à la dose de 1 pour mille, particulièrement pour les traitements en bacs aérés.

On utilise les produits commerciaux classiques tels :

- Les Moussex de PROTEX (922 - 935 GL - 978 GE)
- Les Rhodorsil de Rhône-Poulenc (416-426)
- Le Déhydran (150) de Henkel.

Cet ajout ne perturbe pas la formulation.

Lorsqu'on respecte les proportions appropriées, dont les limites sont définies ci-dessus, les compositions liquides sont stables, homogènes et transparentes dans le domaine de température indiqué plus haut. Elles présentent cette particularité que, si l'on s'écarte du domaine de température de stabilité, les particules, qui se séparent alors, se réémulsionnent quand la température est ramenée au domaine de stabilité, on s'en rend compte par le fait que le liquide redevient transparent.

Pour les administrations parentérales, il est préférable d'utiliser des compositions dont la viscosité est inférieure à 65 cp à 37°C ;les viscosités préférées se situent entre 25 et 60 cp à 37°C.

La préparation d'une composition suivant l'invention comprend de préférence la dissolution préalable du bithionol sulfoxyde dans la quantité appropriée de solvant. Ce dernier doit être choisi parmi les liquides admissibles en pharmacie vétérinaire. Tels sont par exemple les composés suivants : diméthylacétamide, N-méthylpyrrolidone, glycérol, alcool benzylique, éthanol, acétone, diméthylsulfoxyde, sulfolane, lactate d'éthyle, esters d'acides gras, par exemple myristate ou laurate d'isopropyle, glycols, tels que éthylène-propylène ou butylène-glycol, éthers de ces glycols, notamment éthyl éther de l'éthylène glycol, du propylène glycol, ou du butylène glycol ; conviennent également les isopropyl éthers ou les butyl éthers correspondants ; diglycols comme diéthylène glycol, et leurs dérivés comme les éthyl propyl isopropyl ou butyl éthers du diéthylène glycol ; esters gras de l'éthylène glycol ou du propylène glycol, comme laurate, myristate, palmitate, oléate, stéarate de l'éthylène glycol ou du propylène glycol ; esters gras des diglycols correspondants ; polyéthylène et polypropylène glycols de différentes masses moléculaires, notamment 200 à 1500 ou plus.

L'énumération qui précède est donnée à titre non limitatif; il est possible d'employer encore d'autres solvants; les solvants préférés étant naturellement ceux dans lesquels la solubilité du bithionol sulfoxyde est la plus élevée.

Les tensioactifs de la composition sont, bien entendu, choisis parmi les agents de surface présentant toute garantie quant à leur emploi en pharmacie vétérinaire ; on peut indiquer à titre d'exemples non limitatifs : esters de sorbitol et leurs dérivés polyoxy éthylénés, les huiles de ricin polyoxy éthylénées, les copolymères bloc oxyde d'éthylène/oxyde de propylène, le lauryl sulfate de sodium, le dioctyl sulfosuccinate de sodium, lécithines d'oeuf ou de soja, etc.

Comme mentionné plus haut, la composition suivant l'invention contient de préférence un neutralisant de l'acidité du bithionol sulfoxyde ; celui-ci doit être compatible avec l'utilisation en pharmacie, lorsque la composition est prévue pour des applications thérapeutiques. Des neutralisants préférés sont des amines non toxiques, notamment monodi- ou triéthanolamine. On peut cependant utiliser d'autres bases minérales ou organiques.

Un adjuvant fort utile, stabilisant, des nouvelles compositions, est un tampon de pH qui peut être constitué par la solution aqueuse de la composition d'un système de sels tampon classiques. Conviennent particulièrement des phosphates ($KH_2PO_4$-$Na_2HPO_4$) ou, selon le cas, des tampons acétique, citrique, formique,lactique, tartrique ou mandélique. La question du pH ayant une grande importance pour la stabilité de la composition,il convient de distinguer entre les cas où le bithionol sulfoxyde est seul ou accompagné d'un chlorhydrate de tétramisole. Dans le premier cas, les pH les plus favorables s'échelonnent de 5 à 9, la concentration en les sels-tampon étant généralement de 0,005 M à 1 M, et principalement entre 0,01 M et 0,1 M. En présence de chlorhydrate de tétramisole ou de levamisole, le pH est compris dans les limites entre 2 et 5. Pour les compositions renfermant à la fois du bithionol sulfoxyde et chlorhydrate de levamisole, le milieu est tamponné, à pH 2 à 5, au moyen d'un des tampons acides mentionnés plus haut, de préférence additionné d'un anesthésique local du type lidocaîne.

Les compositions suivant l'invention peuvent être stérilisées, avant leur emploi, par chauffage à une température suffisamment élevée ; en effet, comme déjà signalé dans la présente description, lorsqu'une séparation s'est produite au sein de la microémulsion,notamment du fait de la stérilisation, la composition initiale, limpide et homogène, se refait d'elle-même lorsque la température est ramenée dans le domaine de stabilité de la composition.

Un avantage, important pour les emplois thérapeutiques, est que, si l'on ajoute une quantité limitée - généralement de 1 à 10% - de la nouvelle composition à une solution de sérum physiologique (9 g NaCl/l), on obtient une solution limpide ou, au moins, une dispersion extrêmement fine de la composition. Il en résulte pour la composition la possibilité de pénétrer les milieux physiologiques, fait très important pour les applications parentérales. Une bonne résorption de la composition injectée est ainsi assurée.

L'invention est illustrée par les exemples non limitatifs qui suivent.

Dans le souci de simplification, certains composants ont été désignés au tableau 1 par leur dénomination commerciale,

dont voici les significations.

| | |
|---|---|
| "Pluriol 9400" | Copolymère bloc oxyde d'éthylène-oxyde de propylène à 40% d'oxyde d'éthylène, commercialisé par la Société BASF. |
| " Pluriol 6800" ou Pluronic F 68 | Copolymère bloc oxyde d'éthylène-oxyde de propylène à 80% d'oxyde d'éthylène, commercialisé par la BASF et Atochem |
| "Cremophor EL" | Huile de ricin éthoxylée vendue par la Société Cremophor EL |
| "Mergital EL 33" | Huile de ricin éthoxylée vendue par la Société Henkel. |

(Voir Tableau 1 à la page suivante).

## TABLEAU 1

| COMPOSANTS | 1 | 2 | 3 | 4 | 5 | 6 | 7 | 8 | 9 |
|---|---|---|---|---|---|---|---|---|---|
| Bithionol sulfoxyde ........ | 15 | 16 | 12 | 10 | 6,2 | 16 | 16 | 16 | 8 |
| Levamisole chlorhdyrate ...... | | | | | | 1,9 | 2 | 3,8 | 1,9 |
| Tetramisole chlorhydrate ...... | | | | | 2,4 | | | | |
| Monoéthyléther du diéthylène glycol .............. | 25,5 | 30 | 32 | 32,5 | 46,2 | 24,1 | 23,9 | 25 | 26 |
| Lactate d'éthyle ........... | 6,0 | | | 6 | | | | 4,2 | 4,8 |
| Diméthylacétamide ........... | | | | | | 21,5 | 22,4 | 6 | |
| Alcool benzylique ........... | 6,0 | | | | | | | | |
| Monoéthanolamine ............ | 2,5 | 2 | 2 | 1,5 | | 1,5 | 1,5 | 3 | 1,2 |
| Triéthanolamine ............. | | 2 | 2 | | | | | | |
| Pluriol 9400 ................ | | 20 | | | | 18 | 16 | | |
| Cremophor EL ................ | | | | 22,5 | 30 | | | | |

**TABLEAU 1 (suite)**

| Composants | 1 | 2 | 3 | 4 | 5 | 6 | 7 | 8 | 9 |
|---|---|---|---|---|---|---|---|---|---|
| Pluronic F 68 ............... | | | 22 | | | | | | |
| Mergital EL 33 ............... | | | | | | | | 25 | 35,2 |
| Pluriol 6800 ................. | 15,0 | | | | | | | | |
| Eau p. injections<br>. avec tampon phosphate<br>0,025 M ............... | 30 | 30 | 30 | 27,5 | | | | | |
| . avec tampon mandelique<br>0,025 M ............... | | | | | 15,2 | 17 | 18,2 | 17 | 22,9 |
| pH ........................... | 7,6 | 7,4 | 8 | | | 2,7 | 4 | 4,8 | 4 |
| Viscosité à 37°C (cp) ........ | 60 | 44 | | 51 | 43 | | | 38 | |

TABLEAU 1 (suite)

| COMPOSANTS | 10 | 11 | 12 | 13 | 14 | 15 | 16 |
|---|---|---|---|---|---|---|---|
| Bithionol sulfoxyde ................ | 16 | 16 | 16 | 16 | 16 | 16 | 16 |
| Levamisole chlorhydrate ............ | 1,95 | 3,2 | 3,2 | 4,3 | 1,95 | 1,95 | 4,7 |
| Lidocaïne ................. ........ | 0,5 | 0,5 | 0,5 | 0,5 | 0,5 | 0,5 | |
| Monoéthyléther du diéthylène glycol ............. ......... | 24,1 | 22,1 | 22,1 | 21 | 24,1 | 24,1 | 20,3 |
| N Méthyl pyrrolidone ............... | | | 25,9 | 25,9 | 25,9 | 23 | 23,6 |
| Diméthylacétamide ................ | 25,9 | 25,9 | | | | | |
| Alcool benzylique .................. | | | | | | | 0,8 |
| Monoéthanolamine .................. | 0,8 | 0,8 | 0,8 | 0,8 | 0,8 | 0,8 | 1 |
| Triéthanolamine .................. | | | | | | | |
| Pluriol 9400 ..................... | 18 | 18 | 18 | 18 | 18 | 18 | 18,2 |
| Cremophor EL ..................... | | | | | | | |
| Pluronic F 68 ..................... | | | | | | | |
| Mergital EL 33 .................... | | | | | | | |
| Pluriol 6800 ...................... | | | | | | | |
| Eau p.injections | | | | | | | |
| . avec tampon phosphate 0,025 M . | | | | | | | |
| . avec tampon mandelique ....... | 12,8 | 13,5 | 13,5 | 13,5 | 12,8 | 15,7 | 15,4 |
| pH ................................ | 2,37 | 2,7 | 2,34 | 2,31 | 2,46 | 2,34 | 2,3 |
| Viscosité à 37°C (cp) .............. | 30,8 | 28,9 | 42,46 | 42,46 | 39,48 | 44,80 | 49 |

Essai d'efficacité et de tolérance

a. - Ruminants

% d'efficacité contre Fasciola hepatica obtenu avec la solution 2 du tableau 1.

TABLEAU 2

| Dose mg/kg \ Voies | Orale | Intra-péritonéale | Intra-musculaire |
|---|---|---|---|
| 20 | 71,8 | 75 | 82,7 |
| 25 | 84,3 | 94 | 91 |
| 30 | 100 | 99,8 | 100 |

Des tests réalisés, aussi bien par injection intramusculaire que par injection intrapéritonéale ou par administration orale, sur plusieurs séries d'animaux, ont montré une excellente tolérance aussi bien locale que générale.
Aucun effet secondaire n'a été observé.

L'efficacité du bithionol sulfoxyde, sous forme de microémulsion a été confirmé au cours de différentes expérimentations sur ruminants. Après infestation artificielle, à l'aide de métacercaires de Fasciola hepatica, on a trouvé des pourcentages d'activité résumés dans le tableau ci-dessus.
A noter que, dans les expérimentations sur le lot de référence reçevant du bithionol sulfoxyde suspension, par voie orale, à la dose de 40 mg/kg, on a trouvé une efficacité de 99,9%. Le terme "efficacité"désigne, comme on le sait, le rapport % $(N-N_t):N$ où N est le nombre de parasites trouvés chez le lot témoin et $N_t$ le nombre de parasites chez le lot traité.

Voir Tableau 3 à la page suivante.

Bithionol sulfoxyde/Levamisole - 30 mg/6 mg/ kg PV

% d'efficacité

obtenu avec la solution 12 du Tableau 1.

TABLEAU 3

| Voies / Parasites | Orale | Intra-péritonéale | Intra-musculaire |
|---|---|---|---|
| Fasciola hepatica | 100 | 99,9 | 100 |
| Ostertagia ostertagi | 99,6 | 99,5 | 99,8 |
| Cooperia ancophora | 98,3 | 99,6 | 99,5 |
| Trichlostrongylus sp. | 99,9 | 100,0 | 99,8 |
| Oesophagostomum sp. | 100,0 | 100,0 | 100,0 |
| Dictyocaulus viviparus | 99,5 | 99,1 | 100,0 |

Des essais réalisés avec la solution 12 : association de Bithionol sulfoxyde et de Levamisole montrent une réelle activité, aussi bien contre Fasciola hepatica que contre Dictyocaulus viviparus et contre les espèces de strongles digestives (Cooperia, Ostertagia, Trichostrongylus).

Par ailleurs, sur les animaux traités, il n'a été observé aucun effet secondaire tant du point de vue local que du point de vue général.

b. - Poissons - Solution 3

L'efficacité de la Solution 3 du Tableau 1 en aquaculture a été démontrée. C'est ainsi qu'à la dose de 30 mg de BTS par litre d'eau, tous les parasites sont tués alors que le poisson supporte parfaitement le traitement.

La solution 3 est active sur les monogènes du poisson et, par exemple, sur le Diplectanum aequens. Elle est tolérée par les poissons couramment élevés en aquiculture comme le Dicentrarchus labrax.

Essais de stabilité

Des échantillons de la composition de l'exemple 9, qui contient 8% de bithionol sulfoxyde et 1,9% de chlorhydrate de levamisole, sont amenés à différents pH et conservés aux différentes températures, comme indiqué au tableau 4 ci-après.

Les pH inférieurs à 7 sont ajustés avec du tampon mandélique (1 M), tandis que les phosphates habituels ont servi (0,025 M) à fixer le pH supérieur à 7.

Dans chaque cas, le dosage des deux corps actifs est effectué aux différentes périodes choisies et les teneurs en ces corps sont notées au tableau 4.

TABLEAU 4

(solution 9)

| | Bithionol sulfoxyde | | | Chlorhydrate de Levamisole | | |
|---|---|---|---|---|---|---|
| | pH 3,5 | 6 | 8 | pH 3,5 | 6 | 8 |
| Après 10 j | | | | | | |
| à 0°C | 8,0 | 8,0 | 8,0 | 1,9 | 1,8 | 1,7 |
| 20°C | 7,9 | 8,0 | 8,0 | 1,9 | 1,7 | 1,6 |
| 37°C | 7,8 | 7,9 | 7,9 | 1,85 | 1,55 | 1,4 |
| Après 5 mois | | | | | | |
| à 0°C | 7,8 | 7,95 | 8,0 | 1,85 | 1,6 | 1,5 |
| 20°C | 7,6 | 7,9 | 7,9 | 1,7 | 1,4 | 1,3 |
| 37°C | 7,3 | 7,8 | 7,8 | 1,65 | 1,3 | 1,25 |

Une étude de stabilité dans le temps a été effectuée avec les différentes formulations du tableau 1.

Les tableaux 5, 6, 7 montrent deux exemples pour les stabilités des solutions n° 2, 6 et 12.

TABLEAU 5

1. STABILITE de la solution 2 (pH 7,4)

| Temps (en mois) | 0 | 2 | 3 | 6 | 12 | 18 | 24 |
|---|---|---|---|---|---|---|---|
| Bithionol sulfoxyde (en %) | 16 | 15,9 | 16,1 | 16 | 15,9 | 16 | 16 |

TABLEAU 6

2. STABILITE de la solution 6 (pH 2,7)

| Temps (en mois) | 0 | 3 | 6 | 12 | 18 |
|---|---|---|---|---|---|
| Bithionol sulfoxyde (en %) | 16 | 16 | 15,9 | 16 | 16 |
| Levamisole chlorhydrate (en %) | 1,9 | 1,9 | 1,85 | 1,85 | 1,9 |

TABLEAU 7

3. STABILITE de la Solution 12 (pH 2,34)

| Temps (en mois) | 0 | 3 | 6 | 12 | 18 |
|---|---|---|---|---|---|
| Bithionol sulfoxyde (en %) | 16 | 15,9 | 15,8 | 16 | 15,9 |
| Levamisole chlorhydrate (en %) | 3,2 | 3,2 | 3,1 | 3,0 | 3,1 |

On voit que le bithionol sulfoxyde se conserve bien aux pH de 6 à 8, et que même à pH 2 à 4 sa conservation est encore tout à fait satisfaisante. Par contre, le chlorhydrate de levamisole tient mal aux pH 6 à 8, d'où obligation de le conserver à des pH plus bas. Le pH 2 à 4 convient pratiquement à ces deux principes actifs.

Revendications

1. Composition anthelminthique et fongicide comprenant le bithionol sulfoxyde en tant que principe actif, caractérisée en ce que ce sulfoxyde se trouve à l'état de microémulsion dans un liquide renfermant de l'eau, du solvant du bithionol sulfoxyde et un ou plusieurs agents tensioactifs.

2. Composition suivant la revendication 1, caractérisée en ce que le solvant et le tensioactif sont choisis parmi ceux dont l'utilisation en thérapeutique animale est admise.

3. Composition suivant la revendication 1 ou 2, caractérisée en ce qu'elle est constituée en poids % de 1 à 25 de bithionol sulfoxyde, 1 à 50 d'un solvant de ce sulfoxyde, 1 à 40 d'agent tensioactif, 1 à 15 d'un neutralisant du sulfoxyde et 5 à 50 d'eau.

4. Composition suivant la revendication 3, caractérisée en ce que l'eau qu'elle contient est tamponnée à un pH de 5 à 9.

5. Composition suivant la revendication 3, caractérisée en ce qu'elle renferme en outre un autre composé actif, anthelminthique, hydrosoluble, en particulier composé thiazolique, notamment le levamisole.

6. Composition suivant la revendication 5, caractérisée en ce que l'autre composé anthelminthique est le chlorhydrate de L(-) levamisole.

7. Composition suivant la revendication 5 ou 6, caractérisée en ce que la teneur en le composé thiazolique, particulièrement en chlorhydrate de levamisole, est comprise entre

0 et 10% en poids et le milieu aqueux est tamponné à un pH de 2 à 4.

8. Composition suivant la revendication 7, caractérisée en ce qu'elle contient en poids % : 5 à 20 de bithionol sulfoxyde, 1 à 8 de chlorhydrate de levamisole ou de tétramisole, 10 à 55 d'un solvant dudit sulfoxyde, 5 à 35 d'agent tensioactif, 3 à 10 d'une alcanolamine, 0 à 1 d'un anesthésique local type lidocaîne et 10 à 40 d'eau tamponnée à un pH de 2 à 4.

9. Composition suivant une des revendications précédentes, utilisable comme remède contre des atteintes d'animaux, particulièrement ovins et bovins, notamment contre la fasciolose et les strongles gastro-intestinaux ou pulmonaires.

10. Composition suivant une des revendications 1 à 8, utile pour la destruction de parasites en aquaculture et dans la pisciculture.

Office européen des brevets

# RAPPORT DE RECHERCHE EUROPEENNE

0209462

Numéro de la demande

EP 86 40 1564

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | NL-C- 111 318 (N.V. NEDERLANDSCHE COMBINATIE VOOR CHEMISCHE INDUSTRIE) * Colonne 1, linges 38-43; colonne 2, exemple II * | 1-4 | A 61 K 9/10<br>A 61 K 31/10<br>A 01 N 25/04 |
| X,Y | AU-B- 527 154 (ICI AUSTRALIA LTD.) * Page 3, ligne 24 - page 5, ligne 26; page 6, lignes 13,14; page 7, ligne 13 - page 10, ligne 7; revendications 1-6,8,16,28-36 * | 1-10 | |
| Y | FR-A-2 346 001 (DICK et al.) * Page 7, exemple XII * | 1-10 | |

DOMAINES TECHNIQUES RECHERCHES (Int. Cl.4)

A 61 K
A 01 N

Le présent rapport de recherche a été établi pour toutes les revendications

| Lieu de la recherche | Date d'achèvement de la recherche | Examinateur |
|---|---|---|
| LA HAYE | 06-10-1986 | BENZ K.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82